# EUROPEAN PATENT APPLICATION

(11) **EP 4 209 208 A1**
(43) Date of publication of application: **12.07.2023**
(21) Application number: 22150416.0
(22) Date of filing: 06.01.2022
(51) Int. Cl.: A61K 9/48, A61L 31/00, A61M 37/00, A61M 31/00, A61M 15/00

(54) **SWALLOWABLE, SELF-EXPANDABLE DRUG DELIVERY DEVICE AND USES THEREOF**

(71) Applicant: ETH Zurich, 8092 Zurich (CH)
(72) Inventor: LEROUX, Jean-Christophe, 8053 Zürich (CH); LUO, Zhi, 8048 Zürich (CH)

(57) **Abstract**

A swallowable, foldable and self-expandable oral drug delivery tubular device, the device: comprising an outer peripheral surface; having an expanded length of about 2 cm to about 6 cm; having an expanded diameter of about 0.45 cm to about 6 cm; and comprising at least one cavity enclosing a drug, the at least one cavity facing outward from the outer peripheral surface of the device.

## Description

### FIELD OF THE DISCLOSURE

The present disclosure relates to a swallowable and self-expandable drug delivery device. More specifically, the present disclosure is concerned with a swallowable and expandable drug delivery tubular device and uses thereof in non-parenteral drug delivery.

### BACKGROUND OF THE DISCLOSURE

Improving the oral absorption of poorly bioavailable drugs, especially of macromolecules such as peptides, proteins and nucleic acids, is a long-standing goal in pharmaceutical sciences. A major challenge is that the gastrointestinal (GI) tract presents various physiological barriers, which hinder the absorption of drug molecules.

Great efforts have therefore been devoted to developing novel non-parenteral drug delivery systems for these macromolecular drugs (M. Sam, D. Brayden. "Formulation strategies to improve the efficacy of intestinal permeation enhancers." Adv. Drug Deliv. Rev (2021): 113925). Success has been scarce with only a few oral formulations for systemic delivery of peptides having entered clinical trials over the past 30 years, resulting in merely a handful reaching the market. The oral bioavailability of most of these drugs remains very low (generally < 1%).

More particularly, various chemical excipients have been explored to protect the macromolecule drugs from degradation and enhance their absorption by transiently disrupting the barrier function of the GI epithelium. Two permeation enhancers, salcaprozate sodium (SNAC) and sodium caprate (C10), are the most investigated examples. However, the relatively low potency, rapid dilution and absorption have made it difficult to maintain high concentrations of these permeation enhancers at the intestinal mucosa. Therefore, typically a high dose of permeation enhancers is given (e.g., 300 mg SNAC to deliver 3 mg of semaglutide) to achieve a low oral bioavailability in the range of 1%. While protease inhibitors (to reduce premature degradation) such as citric acid have been generally recognized as safe, an important drawback of other inhibitors is the impaired digestion of dietary proteins and other possible side effects such as systemic intoxication and pancreas hypertrophy (Krauland et al. 2004; Melmed et al., 1976).

Various techniques exploiting the physical forces to overcome the mucosal barriers have also been tested. These devices rely on magnetic, mechanical, electrochemical, and acoustic forces to enhance absorption. Currently most of these methods are still in the proof-of-concept and pre-clinical stages. There are several challenges that need to be addressed before their clinical translation, such as safety, efficacy, robustness, and costs. Several attempts have utilized microneedle-equipped devices for the delivery of macromolecules in the GI tract. The so called "robotic pills" are typically composed of an actuation device equipped with microneedles that directly puncture the GI tract mucosa and deliver macromolecular drugs. The complexity of the system, robustness and long-term safety of this technology requires more in-depth investigation before their successful translation. Such systems are also associated with a high variability as the injections can be unsuccessful (Dhalla et al., 2021). Microfabricated devices with dispersible micrometer sized drug reservoirs have been developed. The idea is that an increased surface area enables higher contact with the intestine allowing localized release of drugs and excipients. However, so far, such technology has not demonstrated satisfying results *in vivo,* likely due to the lack of retention and proper orientation of the microcontainers in the mucus layer. The microcontainers have also been integrated onto an elastic foil structure to address the orientation issue. However, the size of the expanded states could not be controlled, and the bioavailability of delivered protein and peptide drugs are below 1% even together with permeation enhancers and protease inhibitors. Another strategy based on superporous hydrogels was proposed to achieve site specific and local release of drugs in the GI tract. The large swelling ratio of these hydrogels enabled the mechanical transient fixation of the drug formulation onto the intestinal wall. A series of *in vitro* and *ex vivo* experiments were performed to correlate the mechanical pressure generated by the hydrogel to the paracellular permeability of different drugs. However, the absorption enhancement effect was not very high (Dorkoosh et al., 2002). It is also unclear how the food contents affect the performance of these swellable formulations. There are several other approaches using physical forces to deliver macromolecular (e.g., peptide/protein) drugs, such as ultrasonication, iontophoresis. However, currently these technologies could not be integrated into a stand-alone swallowable capsule and rely on external devices, which would likely reduce patient compliance and increase the costs.

As a result, novel and more efficient non-parenteral drug delivery strategies are of great interest to the pharmaceutical industry.

There is a need for non-invasive (e.g., needle free) non-parenteral drug administration systems for enhancing systemic delivery, particularly of macromolecular drugs.

The present description refers to a number of documents, the content of which is herein incorporated by reference in their entirety.

### SUMMARY OF THE DISCLOSURE

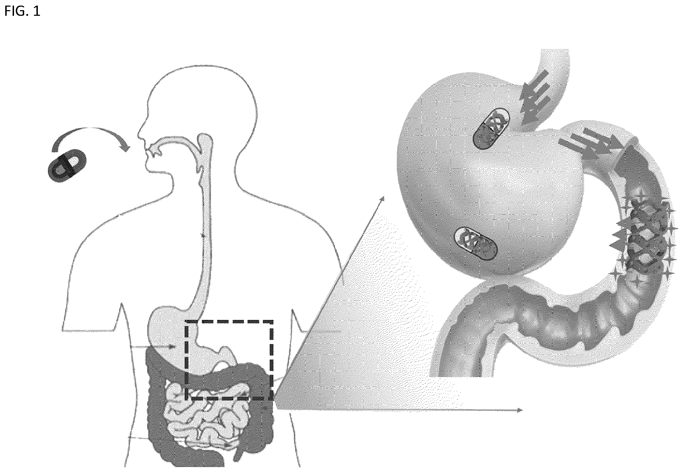

The present disclosure presents a swallowable device designed to release drugs in close proximity to the intestinal wall (or touching it). This novel device comprises a foldable and expandable, optionally degradable tubular structure, with a drug casted into compartments located along external surfaces of the device. The device is folded into an enteric capsule, which selectively releases the device in the GI tract (e.g., small intestine). There, the device expands both radially and axially into its original shape, adapting to the diameter of the GI tract and exposing the drug to the intestinal epithelium (FIG. 1). The physical interaction allows the targeted drug release and the establishment of a sharp local drug concentration gradient, which facilitates its diffusion through the intestinal absorptive barrier.

### Tubular device

### Tubular device shape

### General tubular device shape

The device is a tubular structure comprising at least one drug-containing cavity (i.e., one (a single) or a plurality/array of at least 2, at least 3, at least 4, at least 5, at least 6, at least 7, at least 8, at least 9 or more cavity(ies)) located on or in (an) outer peripheral surface(s) of the tubular structure. The at least one cavity being located on or in an outer peripheral surface of the device so that, when after the device is orally administered to the subject, and the device is coaxially positioned in the GI tract (e.g., in the intestine), the at least one cavity's opening faces the small intestine epithelium and touches it or is in close proximity thereto. When the device comprises more than one cavity, cavities can be spaced over the device's outer peripheral surface.

Devices of the present disclosure can comprise a simple foldable tubular (or hollow polygonal prism) mesh, or a generally tubular structure comprising a central tube (meshed or solid) or rod from which foldable guiding members (e.g., wings, hollow elliptic or round cylinders or hollow prisms) radially extend outward, along the length of the tube or rod. The device's diameter and its length are adapted to guide/position the device coaxially in the GI tract, and the at least one cavity is located on the device's outer peripheral surface.

The term "tubular structure" or "generally tubular structure" is meant to convey herein that the device is generally tubular but is not necessary a cylinder *per se.* Without being so limited, it may take the form of a cylinder/tube or a hollow polygonal prism comprising at least 4 faces (or 5, 6, 7, 8, 9 or more faces) along the device's length, the generally cylindrical or polygonal prism shape of the device being formed either by (1) a single foldable tubular or hollow polygonal prism; or by (2) the combination of the foldable guiding members radially extending from a central tube/rod. It is also meant to convey that the outer peripheral surface, if the device is observed from a top view (or bottom view) may form a circle or a polygon comprising at least 4 sides (or 5, 6, 7, 8, 9 or more sides). For example, if the device comprises 5 foldable guiding members, and if the outer peripheral surfaces of each of the foldable guiding members is flat (instead of being convex), then the device forms a pentagonal prism. This pentagonal prism is generally hollow although it comprises partitions formed by the sides (i.e., ten sides) of the foldable guiding members radially extending outward from the central tube or rod, and although it also comprises a central tube or rod. Within the present disclosure, such shape is referred to as a tubular structure.

In embodiments where the device consists of a simple foldable tube (or hollow polygonal prism), the device's outer peripheral surface is the tube's external wall.

In embodiments where the device comprises foldable guiding members (e.g., wings, hollow elliptic or round cylinders or hollow prisms) radially extending from a central tube or rod, each foldable guiding member (each being independently meshed or solid) comprises an outer peripheral surface. The combined outer peripheral surfaces of each foldable guiding member of the device form the device's outer peripheral surface.

In specific embodiments, foldable guiding members radially extending outward from a tube consist of wings radially extending outward from a central tube/rod, the free end of each wing being shaped so as to form a surface facing the GI epithelium (an outer peripheral surface). More particularly, the shape of the wing's free end can be folded so as to form a surface facing the GI epithelium (an outer peripheral surface), or form a T-shape, the horizontal bar of which forms a surface facing the GI epithelium (an outer peripheral surface). In specific embodiments, foldable guiding members radially extending from a central tube/rod consist of hollow elliptic or round cylinders or hollow prisms (e.g., trapezoidal prisms). In embodiments where the foldable guiding members are hollow prisms, two sides (e.g., non-parallel) of each prism radially extend outward from the central tube/rod, the central tube/rod surface between these two sides forming a third side of the trapezoidal prism, and a fourth side forming an outer peripheral surface. The outer peripheral surface of each prism can be flat or convex (or be subdivided into multiple sides forming a generally convex shape) so as to touch or be in close proximity with the GI tract epithelium. In specific embodiments, the plurality of guiding members radially extending outward from a tube/or are different and may comprise a combination of wings with free end folded and/or T-shaped, hollow elliptic cylinders, hollow round cylinders and/or hollow prisms. In embodiments of the device comprising foldable guiding members (e.g., wings, hollow cylinders or prisms) radially extending from a central tube or rod, outer peripheral surfaces of foldable guiding members contain drug cavities.

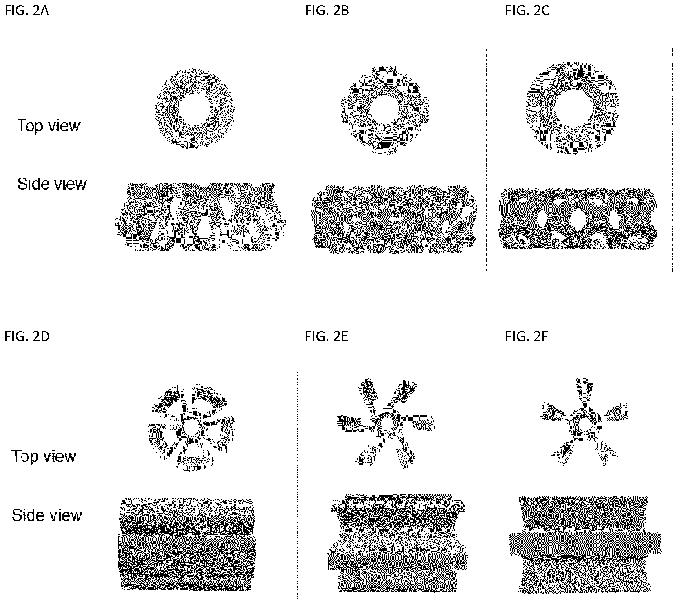

Exemplary devices of the present disclosure are shown comprising a simple tubular mesh (See FIGs. 2A-C for example), or a tubular structure comprising a central tube or rod (e.g., meshed or not) from which foldable guiding members radially extend (e.g., wings) (see FIGs. 2E-2F for example) and hollow prisms (see hollow trapezoidal prisms in FIG. 2D for example).

The thickness of the device material is designed to enable its easy folding. Further, its thickness should be small enough to allow food and fluid transit. In a specific embodiment, the device material has a thickness of about 0.5 mm to about 4 mm.

### Drug cavities and holes

The at least one drug cavity of the device has a volume and is in fluid communication with at least one opening (e.g., cup-shaped) to the outside. In devices containing a plurality of cavities, the term "total cavity volume" refers to the combined volumes of all the device cavities. The total cavity volume is of a size sufficient to contain the drug formulation (and any used excipient) intended for delivery.

At least one, and up to all cavities in the device contain(s) a drug formulation. If the device contains two or more cavities that contain a drug formulation, the drug formulation may be identical or different in the two or more cavities.

The drug cavity(s) is (are) designed so as to accommodate the desired amount of drug and excipient(s) and/or carrier(s). Each drug has specific delivery requirements (e.g., faster or slower release). The present disclosure encompasses the use of cavity sizes, shapes and locations to tailor different drug deliveries.

As indicated above, drug cavities can be located on various parts of the outer peripheral surface of the device. Without being so limited, in specific embodiments, they can be spaced around the device's outer peripheral surface and/or along its length, including on the outer peripheral surfaces of one or more of the foldable guiding members.

The drug cavities can optionally be spaced by holes in the outer peripheral surface (meshed outer peripheral surface). Drug cavities and holes can be distributed in any sequence on the device's outer peripheral surface

(e.g., a line of 1 cavity, 1 hole; 1 cavity, 2 holes; 2 cavities, 1 hole; 2 cavities, 2 holes; etc.; a drug cavity surrounded by 4 holes, etc.). The drug cavities can have any shape. Without being so limited, they can be cup-shaped (e.g., FIGs. 2A-D and F), hemispherical, ellipsoid, cubic, cuboid, conic or pyramidal (the point constituting the bottom of the cavity), cylindric/tubular (FIG. 2E), polyhedrons (5, 6, 7, 8, 9, 10 sides, or more), shaped as channels (e.g., FIG. 2C), or irregularly shaped. The concave portion of the cavities can be located completely or partially below the device's outer peripheral surface, with openings on or above the device's outer peripheral surface (see e.g., FIGs. 2A-D and F). The concave portion of the cavities can alternatively be partially or completely above the device's outer peripheral surface (see e.g., FIG. 2B). In specific embodiments, devices of the present disclosure cab be meshed. The holes forming the mesh structure can have any shape. Without being so limited, the holes can be round (e.g., FIG. 2B), semi-circular, oval or diamond-shaped (e.g., FIGs. 2A and 2C), square, rectangular, trapezoid, triangular, polygonal (with 5, 6, 7, 8, 9, 10 sides, or more), shaped as channels (e.g., FIG. 2C), or irregularly shaped. Each device can comprise a combination of different cavities and, eventually, holes.

The foregoing features are referred to herein as the device shape.

The device shape largely contributes to the device's proper positioning in the intestine and the proper delivery of the drug through the GI tract epithelium.

### Tubular device size

### General device size

The device has a size small enough to fit, when folded, in a standard drug capsule, and large enough to contain the desired drug formulation amount, and, when released from the capsule at the targeted location in the GI tract (e.g., human small intestine), to expand to a size that enables it to be positioned coaxially in the intestine (i.e., with the longitudinal axes of the device and the intestine parallel to each other) (FIG. 1).

The presence of voids in the generally tubular structure of the device of the present invention (see FIGs. 2A-F) allows it to have a volume sufficiently small, when folded, to fit in the capsule. This void can take the form of mesh in the tube wall, of mesh in the foldable guiding elements, of the lumen of tubes and of foldable guiding elements, etc.

Generally, the diameter of the small intestine of a healthy adult ranges between 2.0 and 3.5 cm, the average for an 8-year-old child is about 2.1 cm, and the average for a 15-year-old child is about 2.3 cm. The average diameter of the small intestine of a healthy medium size dog or a similar sized animal is between 0.8 and 1.5 cm. The diameter of standard two-piece capsules used for oral drugs ranges from -0.5 to 1 cm, and their locked length (i.e., when the two pieces are assembled) ranges from ~1.2 to 2.8 cm, preferably from about 2 cm to about 2.8 cm, more preferably 2.1 cm to about 2.8 cm. The capsules internal volume ranges between about 0.13 to about 1.4 mL, preferably from about 0.4 mL to about 1.4 mL.

Without being so limited, the device's folded volume ranges from about 0.13 mL to about 1.4 mL, preferably from about 0.4 mL to about 1.4 mL

Without being so limited, the device's expanded diameter (from its axis to its structural element that is the furthest away from its axis (i.e., the device's outer peripheral surface or, the case being, the opening rims of drug cavities erected above the device's outer peripheral surface) is from about 0.45 cm to about 6 cm. When the device is used for a human adult or child, the device's diameter preferably ranges from about 1.1 cm to about 6 cm. When the device is used for a medium size dogs or other similar sized animals, the device's diameter preferably ranges from 0.45 cm to 2.4 cm.

In specific embodiment, the device's expanded diameter is between about 0.45 cm to about 6 cm, about 0.45 cm to 2.4 cm, about 0.8 cm to about 6 cm, about 0.9 cm to about 6 cm, about 1 cm to about 6 cm, about 1.1 cm to about 6 cm, about 1.2 cm to about 6 cm, about 1.3 cm to about 6 cm, about 1.4 cm to about 6 cm, about 1.5 cm to about 6 cm, about 2 cm to about 6 cm, about 2.5 cm to about 6 cm, about 3 cm to about 6 cm, about 3.5 cm to about 6 cm, about 4 cm to about 6 cm, or about 4.5 cm to about 6 cm; about 1 cm to about 5 cm, about 1.1 cm to about 5 cm, about 1.2 cm to about 5 cm, about 1.3 cm to about 5 cm, about 1.4 cm to about 5 cm, about 1.5 cm to about 5 cm, about 2 cm to about 5 cm, about 2.5 cm to about 5 cm, about 3 cm to about 5 cm, about 3.5 cm to about 5 cm, or about 4 cm to about 5 cm; about 1 cm to about 4.5 cm, about 1.1 cm to about 4.5 cm, about 1.2 cm to about 4.5 cm, about 1.3 cm to about 4.5 cm, about 1.4 cm to about 4.5 cm, about 1.5 cm to about 4.5 cm, about 2 cm to about 4.5 cm, about 2.5 cm to about 4.5 cm, about 3 cm to about 4.5 cm, about 3.5 cm to about 4.5 cm, about 1.1 cm to about 4 cm, about 1.2 cm to about 4 cm, about 1.3 cm to about 4 cm, about 1.4 cm to about 4 cm, about 1.5 cm to about 4 cm, about 2 cm to about 4 cm, about 2.5 cm to about 4 cm, about 3 cm to about 4 cm, or about 3.5 cm to about 4 cm; about 1 cm to about 3.5 cm, about 1.1 cm to about 3.5 cm, about 1.2 cm to about 3.5 cm, about 1.3 cm to about 3.5 cm, about 1.4 cm to about 3.5 cm, about 1.5 cm to about 3.5 cm, about 2 cm to about 3.5 cm, about 2.5 cm to about 3.5 cm, or about 3 cm to about 3.5 cm; about 1 cm to about 3 cm, about 1.1 cm to about 3 cm, about 1.2 cm to about 3 cm, about 1.3 cm to about 3 cm, about 1.4 cm to about 3 cm, about 1.5 cm to about 3 cm, about 2 to about 3 cm, about 2.5 cm to about 3 cm, etc.).

Without being so limited, the ratio of the diameter of the expanded device over the subject's small intestine diameter ranges from about 0.3 to about 3. In a more specific embodiment, it is from about 0.7-1.3.

Without being so limited, the device's expanded length is from about 2 cm to about 6 cm. Without being so limited, the device's expanded "lumen" is from about 0.4 cm to about 4.5 cm. When the device is a simple foldable tubular mesh, its lumen diameter, corresponds to the tube's lumen diameter. When the device comprises foldable guiding members (e.g., wings, hollow prisms) radially extending from a central tube or rod, its "lumen" diameter corresponds to the diameter from the internal wall of its outer peripheral surface to the external wall of central tube/rod. Advantageously, the expanded state diameter and size is known and controlled.

The dimension of the device can be easily adjusted according to the drug dose and patient needs. The dimension of each feature could be adjusted within a range. For example, if a single tubular mesh is used e.g., as shown in FIGs. 2A-C, a wall thickness of about 0.5 mm - 4 mm can be used.

### Drug cavity sizes

The tunable drug cavities are sized so as to allow a simple drug loading step such as coating (e.g., dip, spray coating), drop casting or to allow insertion of minitablets therein (e.g., diameter of 1 mm-3 mm) and/or to allow delivery over a period of between 5 minutes up to 6 hours.

Each cavity can have a volume of at least 10 nL, at least 20 nL, at least 30 nL, at least 40 nL, at least 50 nL, at least 60 nL, at least 70 nL, at least 80 nL, at least 90 nL; at least 0.1 µL, at least 0.2 µL, at least 0.5 µL, at least 1 µL, at least 5 µL, at least 10 µL, at least 20 µL, at least 30 µL, at least 50 µL, at least 60 µL, at least 70 µL and up to 100 µL; between 10 nL and 100 µL; or between 20 nL and 100 µL; or between 30 nL and 100 µL; or between 40 nL and 100 µL; or between 50 nL and 100 µL; or between 60 nL and 100 µL; or between 70 nL and 100 µL; or between 80 nL and 100 µL; or between 90 nL and 100 µL; or between 0.1 µL and 100 µL; or between 0.2 µL and 100 µL; or between 0.5 µL and 100 µL; or between 1 µL and 100 µL; or between 5 µL and 100 µL; or between 10 µL and 100 µL; or between 20 µL and 100 µL; or between 30 µL and 100 µL; or between 50 µL and 100 µL; or between 60 µL and 100 µL; or between 70 µL and 100 µL. The device can comprise a combination of cavities having different sizes as listed above or within the stated ranges.

### Device elasticity

Elasticity is the ability of a body to resist a distorting influence and to return to its original size and shape when that influence or force is removed. The device's foldable elements' elasticity enables it to be folded and inserted into a capsule, and once released from the capsule in the GI tract, enables it to self expand to regain its original size and shape (expanded state). The device has an elasticity that enables its foldability and self-expandability.

In specific embodiments, the device material has a Young's modulus of about 0.01 MPa to about 1000 MPa (about 0.1, 0.2, 0.5, 1, 2, 3, 4, 5, 6, 7, 10, 15, 20, 30, 40, 50, 75 MPa, to about 50, 100, 200, 300, 400, 500, 600, 700, 800 or 900 MPa) at a temperature of between about 20°C and about 55°C.

### Device material

The material of the device pharmaceutically acceptable. It is also sufficiently flexible/elastic to allow for its easy mechanical folding and expanding. The device material comprises or consists of an elastomer. The device material is such as to confer it has Young's modulus of about 0.01 MPa to about 1000 MPa. Without being so limited, materials encompassed by the present disclosure include elastomers e.g., polyester-based elastomers, such as poly(ε-caprolactone), poly(glycolide), poly(lactide), poly(hydroxyalkanoate), poly(4-hydroxybutyrate), poly(dioxanone), poly(1,3-trimethylene carbonate), poly(ethylene succinate), polybutylene terephthalate, and their copolymers; polyether-based elastomers, such as poly(ethylene glycol), poly(tetramethylene) glycol, poly(propylene oxide), poly(p-phenylene oxide), polyaryletherketone, polyether ether ketone, polyphenyl ether, polytetrahydrofuran, and their copolymers; polyamides-based elastomers; polyurethanes-based elastomers; poly(ester amide)-based elastomers; polysaccharides-based elastomers; poly(β-thioester) and poly(β-amino ester)-based elastomers; polydimethylsiloxane (PDMS)-based elastomers; polyacrylates-based elastomers; synthetic and natural rubbers such as polybutadiene; copolymers (block, diblock, or triblock) of at least two thereof; or a mixture of at least two of these materials. In specific embodiments, the material is non biodegradable (e.g., polyether-based elastomers; polydimethylsiloxane (PDMS)-based elastomers; polyacrylates-based elastomers; synthetic and natural rubbers such as polybutadiene). In other specific embodiments, the material is biodegradable (e.g., polyester-based elastomers, polyamides-based elastomers; polyurethanes-based elastomers; poly(ester amide)-based elastomers; polysaccharides-based elastomers; poly(β-thioester) and poly(β-amino ester)-based elastomers). While these polymers will be chemically functionalized to form crosslinked network, the main chain of this polymers could be degraded under different mechanisms such as hydrolysis, reverse reaction, chemical cleavage, enzymatic degradation, etc. The degradation kinetics could also be adjusted from a few hours to a few days under the intestinal fluid conditions.

The device can be made of a single material (i.e., the material of foldable parts thereof) or multiple materials, e.g., a first material for foldable parts thereof (single tube or foldable guiding members) and a second material for the central tube or rod. The device material(s) may be biodegradable or nonbiodegradable. In a more specific embodiment, it is biodegradable. Degradable elastomers: certain polymers degrade under physiological conditions with tunable kinetics. They minimize risks associated with congestion in the GI tract since the device is degradable. In specific embodiments, elastomeric materials based on polyesters, or poly(β-amino ester)s, which are compatible with both 3D printing and molding fabrication methods are used.

In specific embodiments, the device may be produced by 3D printing and molding.

### Capsule material

Any pharmaceutically acceptable capsule can be used in for devices of the present disclosure. The capsule can advantageously be enteric to enable its disintegration in the desired location of the GI tract, e.g., the small intestine.

Without being so limited, capsules that can be used for the present disclosure can be made from gelatin, hydroxypropylmethyl cellulose or pullulan and their derivatives. The capsules can be intrinsically enteric or coated with an enteric polymer such as methacrylic acid copolymers, cellulose acetate phthalate or hydroxypropylmethylcellulose phthalate, or polyvinyl acetate phthalate. As used herein the term "enteric capsule" refers to a capsule made from an enteric polymer or and/or a capsule coated with an enteric coating.

### Drug

Drug(s) as used herein refers to any pharmacologically active molecule or mixture of molecules (including non approved active pharmaceutical ingredients (APIs)). As used herein, mixtures of molecules display pharmacological activity. Without being so limited, biological extracts such as plant or animal extracts are encompassed by the term mixture of molecules, and thereby by the term drug. Drugs that can be incorporated into the device of the present disclosure can be low molecular weight active pharmaceutical ingredients (< 1000 g/mol) or large molecules (>1000 g/mol) such as polysaccharides, peptides, proteins, nucleic acids, dendrimers, polymer-drug conjugates, proteolysis-targeting chimeras (PROTACs); Lysosome-targeting chimaeras (LYTAC); antibody-based PROTACs (AbTAC), antibody-drug conjugates, lipid or polymeric nanoparticles, exosomes, and vaccine antigens. While devices of the present disclosure are most advantageously used to deliver drugs with high molecular weight (without being so limited, e.g., between 1kDA and 50 kDa; e.g., about 1 to about 45 kDa) or size (without being so limited between 0.5 nm and 10 nm; or between about 0.5 nm and 6 nm) as such drugs typically display low oral absorption and require injection, there are no lower size limitations to drugs that can be used in the devices of the present disclosure. The devices of the disclosure would also advantageously be used for drugs displaying low oral bioavailability related to degradation in the GI tract and/or poor diffusibility (poor absorption) across the mucosa, including drugs smaller than 1 kDa, and including cannabis oils including e.g., cannabidiol and/or cannabigerol and/or tetrahydrocannabinol.

Drugs that would particularly benefit from the delivery device of the present disclosure include the following: (1) semaglutide, an anti-diabetic and anti-obesity medication that acts like human glucagon-like peptide-1. The drug has a molecular weight of 4113 Da with an oral bioavailability -1% even in the presence of large amount permeation enhancers as excipients. The recently approved oral tablet (Rybelsus) from Novo Nordisk for diabetes) also requires the drug to be taken at a specific time before breakfast every day, as the absorption is affected by food in the stomach. (2) octreotride: has an oral bioavailability of 0.5-1% even in the presence of permeation enhancers; (3) human growth hormone, a drug to treat children's growth disorders and adult growth hormone deficiency. As is typical to protein drugs with a high molecular weight (22 kDa) and being prone to be degraded in the GI tract, there are currently no oral formulations of this drug on the market; (4) insulin is currently only available for injection or pulmonary administration; (4) TNF-alpha inhibitors are antibodies that need to be injected. Other peptide and protein (macromolecular drugs) drugs with low oral bioavailability and high sensitivity to degradation in the GI-tract: (5) PROTACs, a new type of drug molecule that can degrade the target pathogenic proteins and regulate the related signaling pathways. With similar molecular weight to peptide-based drugs, the PROTACs often have low oral bioavailability. The device could avoid several modifications to achieve stability and absorption via the GI tract; and (5) vaccines: As injected vaccines only trigger systemic immunization; mucosal immunity could also or alternatively be stimulated via the transmucosal route.

In view of the size limitation of the device, and consequently limited drug compartment(s) size, the device may be used to deliver drugs that can usefully be administered at a dose lower than about 100 mg (or lower than about 50 mg).

### Drug formulation

The drug(s) can be loaded in the device with at least one excipient (one or more excipient(s)).

Without being so limited, excipients/carriers can be loaded inside one or more compartment(s) (cavities) of the device, included as filler in the capsule enclosing the device and/or coated on the capsule.

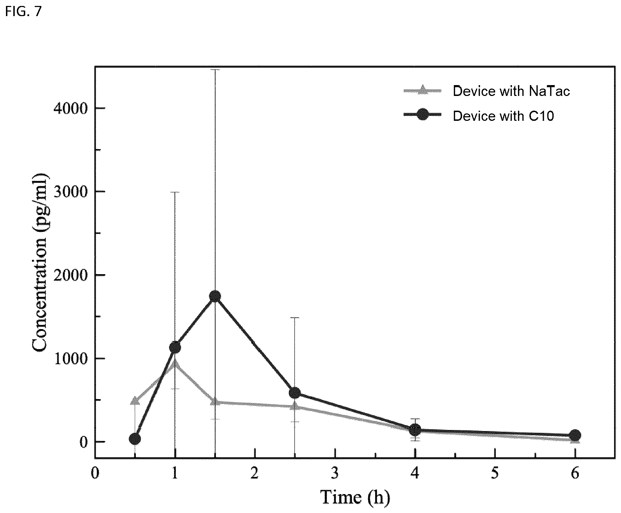

Without being so limited, the following excipients can be used:
Enteric coating: In order to release the drug delivery device in the intestine, unless a capsule made of an enteric polymer is used, enteric coating avoids premature opening of the capsules in the stomach. Furthermore, by tuning the thickness of the coating layer as well as the pH sensitivity of the enteric polymer, it is also possible to control the time and location of the release. As indicated above, the enteric material can be coated on capsules enclosing the device of the present disclosure.
*Polymeric and lipidic excipients:* The drug can be mixed with one or more polymeric/lipidic excipients in order to control its release kinetics in the GI tract. Without being so limited, these excipients can be poly(vinyl alcohol) (PVA), poly(N-vinylpyrrolidone) (PVP), poly(ethylene glycol) (PEG), polycarbophil, Starch and derivatives, cellulose derivatives, Chitosan, Carbomer, Alginate and derivatives fatty acids, and waxes.
*Enzymatic inhibitors:* The degradation of drug compounds due to enzymatic reactions is also one of the barriers in the oral delivery, especially for peptide and protein-based drugs. One or more enzymatic inhibitor(s) can be incorporated inside one or more cavity (with or separately from the drug(s)) on the external surface of the tube, or inside the capsule with the device.
*Permeation enhancers:* They are compounds capable of enhancing membrane permeability in the GI tract. They function with different mechanisms of actions, e.g., by disrupting the mucus layer, transiently opening tight junctions, or fluidizing the epithelial cell membranes. The combination of the device with the chemical disruption produced by a permeation enhancer can further promote the diffusion of the drug (e.g., hydrophilic macromolecules) (see e.g., FIG. 7).

As used herein, the permeation enhancers that can be used in the device or capsule include without being so limited, salcaprozate sodium (SNAC); fatty acids and derivatives, such as sodium caprate (C10), sodium caprylate, and palmitoylcarnitine; bile salts and other steroidal detergents, such as deoxycholate, sodium taurocholate (NaTaC), glycocholate, and saponin; natural and synthetic surfactants, such as phospholipids, sodium lauryl sulphate, dioctyl sodium sulfosuccinate, and derivatives thereof; chelators such as EDTA, citric acid/citrate, and polyacrylates; positively charged polymers such as chitosan; cyclodextrins and derivatives; laurocapram and derivatives; thiomenthol and derivatives; and polysorbates and derivatives.

*Protease inhibitors:* A major obstacle to the delivery of peptides is their susceptibility to enzymatic degradation. Protease inhibitors that can inactivate luminal and/or brush border enzymes have therefore been proposed as the excipient for oral drug delivery. Protease inhibitors that can be used in the device include without being so limited, citric acid, aprotinin, Bowman-Birk inhibitor; bacitracin and derivatives; N-acetyl-cysteine; soybean trypsin inhibitor; camostat mesylate; sodium glycocholate; hydroxyethylcellulose; and poly(acrylic acids).

If the device includes two or more drug cavities including a drug formulation, the two or more drug formulations may be identical or different.

### Methods of use

The present disclosure provides for the use of a swallowable and self-expandable drug delivery described herein for the non-parenteral systemic delivery of a drug in a subject in need thereof. The device is enclosed in an enteric capsule to ensure targeted drug delivery in the intestine. The capsule follows normal transit and once it reaches its destination in the intestine, it releases the device, and optional filler comprising one or more excipients. The device then self-expands and positions itself coaxially in the intestine. The device then follows the normal transit while releasing the at least one drug(s) and excipient(s) that it contains, towards and in close proximity to the intestinal epithelium or touching it. A steep drug concentration gradient can thus be established and maintained to achieve better drug permeation.

The delivery of the drug from the device through the subject's intestinal epithelium and into the subject's systemic circulation (via lymphatic vessels and/or blood vessels) occurs through passive migration of the drug if the latter is not linked to a ligand capable of recognizing a receptor at the surface of the epithelial mucosa. Since after the device release from the capsule, the at least one drug loaded cavity faces the intestinal epithelium and touches or is in close proximity thereto throughout the device transit, the drug release towards the epithelium is always favored ("unidirectional release"). According to the present disclosure, the device is dynamic (i.e., moves with normal transit once orally administered and throughout drug delivery). It is a needle free, electricity free delivery that does not involve physical piercing of the epithelium.

### Combination therapies

The device of the present disclosure can be applied as a stand-alone delivery means for drugs or in combination with other drug delivery strategies, such as permeation enhancers as described above.

### Route

The device is for oral delivery.

### Subject

As used herein the term "subject" is meant to refer to any animal, such as a mammal, including a human, dog, cat, pig, cow, monkey, cattle, horse, etc. In a particular embodiment, it refers to a human, in specific embodiments to an adult subject.

### Device manufacturing method

The device of the present disclosure can be designed using common Computer Aided Design (CAD) softwares, such as AutoCAD^{™}, Solidworks^{™}, etc. The devices can be produced using any known method, including but no limited to 3D printing with techniques such as digital light processing (DLP), carving, molding (e.g., compression molding, mold casting, and injection molding), which enables rapid prototyping, optimization, as well as mass production.

### Drug loading

Any known drug loading method can be used including but not limited to coating (e.g., dip, spray coating), drop casting, paste injection, mini-tablet loading (e.g., diameter of 1 mm-3 mm), etc. With the coating method, the drugs and excipients are dissolved or dispersed in a suitable solvent in the absence of presence of other excipients (e.g., lipids, polymers), which is then evaporated to leave a solid matrix inside the patch. In the paste injection method, a semi-solid solution or dispersion of the drug and excipients is deposited by extrusion in the device. When the drug is admixed with lipids, the latter can be dispersed/dissolved in a lipidic matrix that is heated above its melting temperature, deposited on the device and cooled down.

### Drug delivery

Once the drug loaded device is loaded in the capsule, and the capsule is swallowed by the patient, the capsule transits to its delivery target location in the GI tract (e.g., small intestine), releases the device, which then unfolds and positions itself coaxially in the small intestine lumen, with drug compartments opening in close proximity to the GI epithelium or touching it. Without being so limited, in Examples presented herein, the drug crosses the epithelium, and reaches the blood and/or lymphatic vessels (systemic circulation).

### Non exhaustive advantages

Self-expandable: the elastomeric materials and the design of the device allows the device to self-expand upon its release from the capsule without the need for any actuation mechanisms, such as chemical reaction or heat.

The aspect ratio (diameter and length) of the device also ensures its correct positioning in the GI tract.

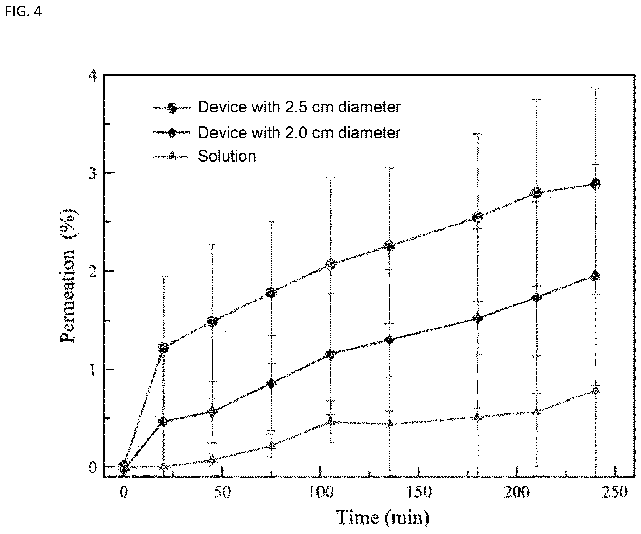

Unidirectional release and sharp drug concentration gradient: Due to the highly dynamic GI tract environment, controlling the release direction of the drug *in vivo* is highly challenging. The expandable device of the present disclosure (tubular structure) promotes the correct orientation of drug towards the intestinal epithelium and thus achieves localized high drug concentration which promotes diffusive flux of the drug. According to Fick law of diffusion, diffusive flux goes from a high-concentration area to a low-concentration area and is proportional to the concentration gradient. *Ex vivo* studies were performed on excised pig intestines (FIG. 4) to examine such effect. A considerable increase in intestinal drug permeation was observed upon application of the expandable device with a diameter comparable to that of the intestine, compared to a smaller device and a solution of the drug.

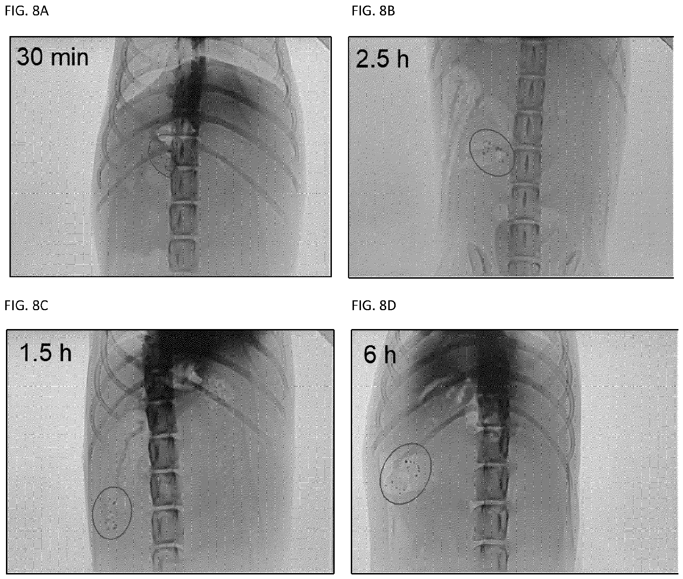

Non-invasive: The expandable device is non-invasive and does not require any strong physical disruption of the intestinal tissue. This may reduce potential safety risks due to the systemic exposure to food antigens and microorganisms. Furthermore, with a tubular structure the device avoids or reduces disturbing the passage of other components in the intestine, such as liquids and foods. Repetitive dosing to beagle dogs for a duration of almost a year has never demonstrated any signs of congestion of the GI tract or other side effects such as weight loss. In fact, *in vivo* Xray imaging (FIG. 8) has shown that the device could transit through the GI tract smoothly and reach colon within six hours after administration.

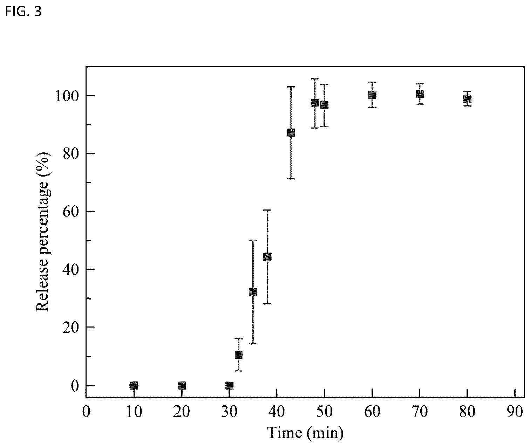

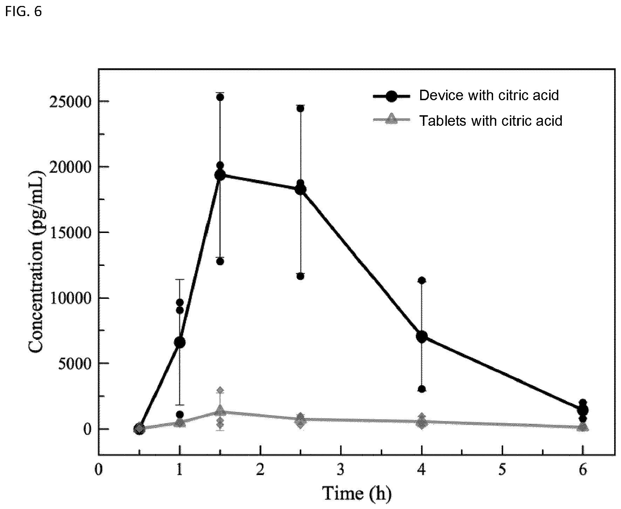

Versatile for different drug formulations: The swallowable device is usable for the delivery of various types of drugs and excipients. Various types of excipients can be loaded in the delivery device together with the drug compounds, such as permeation enhancers (e.g., polymer matrix) (e.g., permeation enhancers such as C10 (e.g., FIG. 7)), polymers, and enzymatic inhibitors (e.g., protease inhibitors such as citric acid (see e.g., FIG. 6)). A controlled release kinetics can thus be achieved (see e.g., FIG. 3). With a tunable drug compartment design, the loaded doses are also easily adjusted.

Robustness: A common disadvantage of the physical mixture of drugs and other excipients is the lack of robustness in achieving high drug concentration near intestinal wall. For example, with the commercial semaglutide drug tablet, the rate of successful delivery is less than 50% for single dosing. It was reported that in the single-dose trial, a high proportion of subjects distributed among all oral semaglutide treatment arms had no measurable semaglutide in plasma (Granhall et al., 2019). In contrast, the expandable device ensures a close contact of the drug and mucosa and thus leads to more reproducible delivery profile.

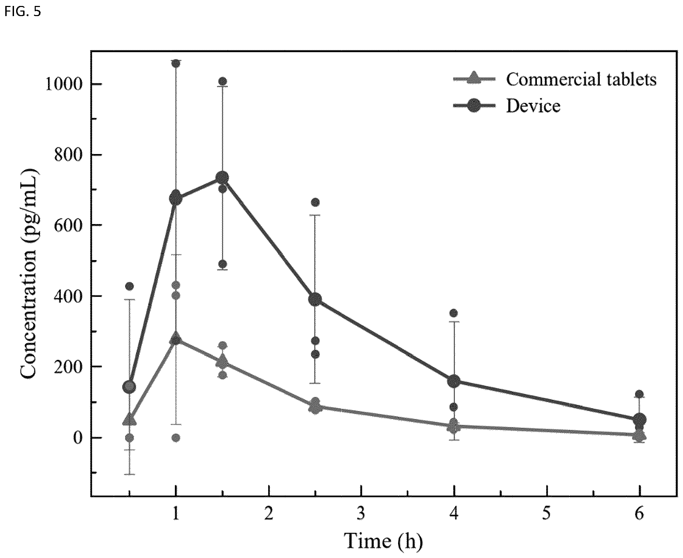

Higher bioavailability: The bioavailability of most oral formulations for macromolecular drugs such as peptides is very low, i.e., 1% or lower. A series of *in vivo* experiment in beagle dog models were performed using desmopressin (Mw 1069 Da, antidiuretic peptide) as the model drug. The self-expandable drug delivery device could achieve ~3.6 times higher bioavailability than commercial tablets (FIG. 5). When combined with citric acid, the optimized formulation led to more than 17 times higher absorption (FIG. 6) than commercial tablets that were administered together with citric acid, or more than 107 times higher compared to commercial tablets. Considering the fact that most of the approved oral peptide drugs have a bioavailability less than 1%, such enhancement is clearly a step-change for the field.

Patient friendliness and reduced health care cost. The patch application is simple and user friendly as no special training or other devices are required. Therefore, drugs that typically must be administered via injections can be pain-free self-administrated by patients, which most likely increases patient acceptance and hence their compliance, especially for children or subjects with antipathy to needles. The fact that the device is self-applicable allows the home administration of drugs, which further increases patient comfort as well as reduces the costs and requirements for medical staff.

Scalability and costs. Due to the simple and scalable manufacturing process by molding, pre-existing manufacturing plants can be used with small adjustments. Moreover, the drug loading processes by e.g., film formation or paste injection, are well established processes and can be easily adapted. As a result, simple manufacturing, low-cost materials and simple implementation in existing manufacturing plants make the technology readily available for small and large companies. For rapid prototyping purpose, the expandable device was fabricated by 3D printing using stereolithography (SLA) and a polymeric resin prepared by Michael Addition. The material presents high elasticity, good resilience to mechanical compression (e.g., to intestinal mechanical forces). A formulation of the model peptide desmopressin, and poly(N-vinylpyrrolidone) (PVP), was incorporated into the cavities on the surface of the device using a solvent-casting evaporation method. The device was then inserted into a gelatin capsule together with citric acids followed by enteric coating of the capsule using Eudragit^{™} L100-55 solution.

More specifically, in accordance with the present disclosure, there are provided the following items:
Item 1. Swallowable, foldable and self-expandable oral drug delivery tubular device, the device:
   comprising an outer peripheral surface;
   having an expanded length of about 2 cm to about 6 cm;
   having an expanded diameter of about 0.45 cm to about 6 cm; and
   comprising at least one cavity enclosing a drug, the at least one cavity facing outward from the outer peripheral surface of the device. In a more specific embodiment, the expanded diameter of the device used for humans is of about 1.1 cm to about 6 cm.
Item 2. The device of item 1, wherein the tubular device comprises a single tubular mesh.
Item 3. The device of item 1, wherein the tubular device comprises foldable guiding members radially extending from a central tube or rod, each foldable guiding member comprising a guiding member outer peripheral surface, the combined guiding member outer peripheral surfaces of all foldable guiding members forming the device's outer peripheral surface.
Item 4. The device of any one of items 1-3, wherein the drug is in a formulation further comprising at least one excipient.
Item 5. The device of item 4, wherein the at least one excipient comprises a permeation enhancer.
Item 6. The device of item 3 or 4, wherein the at least one excipient comprises a protease inhibitor.
Item 7. The device of any one of items 1-6, wherein the drug has a molecular weight of about 1 kDa to about 50 kDa.
Item 8. The device of any one of items 1-6, comprising drug cavities having a volume between 10 nL and 100 µL.
Item 9. Enteric capsule enclosing the device defined in any one of items 1-8.
Item 10. The capsule of item 9, further comprising a further excipient.
Item 11. The device of any one of items 1-8 or the capsule of item 9 or 10, for oral administration of the drug to a subject.
Item 12. The device of capsule of item 11, wherein the subject is a human and the device's expanded diameter of about 1.1 cm to about 6 cm.
Item 13. A use of the device as defined in any one of items 1 to 8 enclosed in an enteric capsule, for the oral administration of the drug to a subject, the use comprising:
   orally administering the capsule to the subject, whereby when the capsule releases the device in the small intestine, the device self-expands and positions itself coaxially in the intestine, and
   whereby the drug migrates through the intestine epithelium and enters systemic circulation of the subject.
Item 14. The use of item 13, wherein the subject is a human.

Other objects, advantages and features of the present disclosure will become more apparent upon reading of the following non-restrictive description of specific embodiments thereof, given by way of example only with reference to the accompanying drawings.

### BRIEF DESCRIPTION OF THE DRAWINGS

In the appended drawings:
FIG. 1: Illustration of an exemplary self-expandable oral drug delivery device operation.
FIGs. 2A-F: Illustrative embodiments of the self-expandable oral drug delivery device.
FIG. 3: *In vitro* drug release in simulated intestinal fluid by the self-expandable oral drug delivery device.
FIG. 4: Comparison of the *ex vivo* effects of two diameter sizes (2 cm and 2.5 cm) of self-expandable oral drug delivery device on transmucosal drug delivery.
FIG. 5: Comparison of the plasma concentration of drug in beagle dogs after administration of a desmopressin loaded self-expandable oral drug delivery device with that of Minirin^{™} tablets.
FIG. 6: Comparison of the plasma concentration of drug in beagle dogs after administration of a desmopressin loaded self-expandable oral drug delivery device in combination with citric acid with that of Minirin^{™} tablets in combination with citric acid.
FIG. 7: Comparison of the plasma concentration of drug in beagle dogs after administration of a desmopressin loaded self-expandable oral drug delivery device in combination with two different permeation enhancers, i.e., C10 and NaTaC.
FIGs. 8A-D: Representative *in vivo* imaging of the device transit through GI tract of beagle dogs.

### DESCRIPTION OF ILLUSTRATIVE EMBODIMENTS

The present disclosure is illustrated in further details by the following non-limiting examples.

### EXAMPLE 1: Structural features and operation of the device of the present disclosure

The structural features of the device of the present disclosure have been designed to fulfill several functional requirements. (1) The interior of the tubular structure is generally hollow so that the device does not affect the function of the GI lumen. (2) The device is also designed to fit, when folded, in a standard drug capsule and to expand, when released from the capsule in the human small intestine, to a size that enables it to be positioned coaxially in the intestine (i.e., with the longitudinal axes of the device and the intestine parallel to each other) (FIG. 1). The average diameter of the small intestine of a healthy adult is between 2.0 and 3.5 cm. The diameter of standard two-piece capsules used for oral drugs ranges from ~0.5 to 1 cm, and their locked length (i.e., when the two pieces are assembled) ranges from ~1.2 to 2.8 cm, preferably 2 cm to 2.8 cm. The capsule internal volume ranges between 0.13 mL to 1.4 mL, preferably 0.4 mL to 1.4 mL. The presence of voids in the generally tubular structure of the device of the present disclosure (see FIGs. 2A-F) allows it to have a volume sufficiently small, i.e., ranging from 0.13 mL to 1.4 mL, preferably 0.4 mL to 1.4 mL, when folded, to fit in the capsule. (3) the device also comprises drugs and excipient compartments/cavities located on external surfaces of the device, i.e., surfaces that are facing the GI tract epithelium to enable the release of the drug and excipients in close proximity to the epithelium or touching the epithelium (outer peripheral surface of the device). The tunable cavities can be used to load various types of drug formulations and excipients.

### EXAMPLE 2: Illustrative 3D structures of the device

The expandable drug delivery device can have various 3D structures provided they have the characteristics listed above. FIGs. 2A-F present specific embodiments of the swallowable self-expandable device of the present disclosure. FIGs. 2A-C present devices that consist of simple tubular meshes comprising drug cavities on its outer peripheral surface (external tubular wall). Their top views show that they are hollow. In FIG. 2A, a side view of the tubular mesh is shown where 6 shallow cup-shaped drug cavities can be observed (i.e., other drug cavities are present on sides of the device that are not visible on the view of FIG. 2A) spaced along the external tubular wall (Device used in Examples 3-8). In FIG. 2B, a side view of the tubular mesh is shown on which about 14 drug cavities carved in relief on the external tubular wall can be observed (i.e., other drug cavities are present on sides of the device that are not visible on the view of FIG. 2B) which spaced along the wall in alternance with 14 holes. In FIG. 2C, a side view of the tubular mesh very similar to that shown in FIG. 2A except that the shallow cup-shaped cavities are joined by drug cavities shaped as channels. FIGs. 2D to 2F present devices comprising foldable guiding members radially extending from a central tube, each foldable guiding member comprising an outer peripheral surface containing drug cavities. In FIG. 2D, a top view of the device shows 5 foldable guiding members in the form of hollow trapezoidal prisms. FIG. 2D shows a side view of this device presenting the outer peripheral surfaces of two of these 5 foldable guiding members. A partial view of a third foldable guiding member in also presented. Three shallow cup-shaped drug cavities are shown of each outer peripheral surface. In the devices presented in FIG. 2E and 2F, the foldable guiding members radially extending from a central tube are wings. In FIG. 2E, a top view of the device shows 6 foldable wings, the free end of each wing being folded so as to form outer peripheral surfaces. In FIG. 2E, a side view of the device shows the outer peripheral surface of one folded wing, comprising 4 shallow cup-shaped drug cavities. This view also partially displays three other wings of the device. In FIG. 2F, a top view of the device shows 5 foldable wings, the free end of each wing T-shaped so as to form outer peripheral surfaces. In FIG. 2E, a side view of the device shows the outer peripheral surface of one T-shape ended wing, comprising 4 shallow cup-shaped drug cavities. This view also partially displays two other wings of the device.

### EXAMPLE 3: Manufacture of the device

To prepare a photopolymerizable resin, a poly(β-thioester) polymer, poly(EDT-co-BDA), was prepared by copolymerizing (ethylenedioxy)diethanethiol (EDT) and 1,4-butanediol dimethacrylate (BDA) yielding a polymer with an averaged molecular weight of 6300 Da. The 9 g of the polymer, a phosphine oxide initiator (0.75 wt%), the photo absorber Sudan I (0.04 wt%), dilution solvent NVP (11 wt%), and UV inhibitor vitamin E (0.3 wt%) were mixed and sonicated at 40 °C until a homogeneous resin was obtained. A commercial DLP 3D printer (Asiga PICO2, Sydney, Australia) was employed to build the 3D objects. The printer comprised a LED light source of 405 nm and a customized tray with a heating system to allow printing at various temperatures e.g., 75 °C. After printing, resin residuals were removed with isopropanol and ethanol. Subsequently, the printed device was cured under 405 nm UV-light for 15 min followed by extensive rinsing with ethanol and drying overnight.

### EXAMPLE 4: Drug loading and In vitro drug release

Standard *in vitro* release studies were performed using USP device type II. To load the drug formulation onto the device, a methanol solution containing 200 mg/mL desmopressin and 400 mg/mL PVP was first prepared. Then 0.1 mL solution was casted dropwise in the device drug cavities followed by drying overnight to remove all the solvent residues. A size 000 gelatin capsule was enteric-coated using Eudragit^{™} L100-55 polymer. The final enteric capsule containing an expanded 8 mm (for a ratio of the diameter of the expanded device to the diameter of small intestine of about 0.5 to about 1) diameter tubular mesh device (FIG. 2A), 20 mg of desmopressin model drug and 40 mg of PVP was first immersed in simulated gastric fluids (SGF, pH = 1.2) for half hour before the solvent was switched to simulated intestinal fluid (SIF, pH = 6.8). The temperature was set at 37 °C, and the rotating speed was at 100 rpm. Aliquots of 1 mL were sampled at the predetermined time points and the sampled volume was replaced with fresh SGF or SIF buffer. Quantification was carried by HPLC (VWR Hitachi Chromaster system with 5160 pump, 5260 autosampler, 5310 column oven and 5430 diode array detector, VWR International, Radnor, PA) with the UV wavelength set at 280 nm. The C18 column (XBridge^{™}, 5 µm, 250 x 4.6 mm, Waters, Milford, MA) was used for the separation, while a mixture of 75 % H₂O (0.1 % v/v trifluoroacetic acid) and 25 % acetonitrile was used for the mobile phase. Results are shown in FIG. 3. It can be seen that at the intestinal pH (after 30 min), desmopressin was released within about 20 minutes. Therefore, with enteric capsules, the device can be protected in the stomach and is only released from the capsule after reaching the intestine. The drug release kinetics is also controlled with the polymeric excipient.

### EXAMPLE 5: Ex vivo studies on the effects of localized drug delivery by the self-expandable oral drug delivery device

*Ex vivo* studies was performed on fresh porcine intestines obtained from the slaughterhouse. Intestinal sacs were prepared containing either a desmopressin solution or a desmopressin-loaded expandable device described in Example 4 except that its diameter was 2.0 cm or 2.5 cm, with the same drug and PVP concentrations as in Example 4 loaded in the capsules described in Example 4. All intestine tissues were incubated in modified DMEM (i.e., the pH of the medium was adjusted to 6.8 with an HCI solution, and its osmolality was within the range of 280-295 mOsm/kg) for 4 h, during which 1 ml sample solutions were withdrawn at predefined timepoints (0, 20, 45, 75, 105, 135, 180, 210 and 240 min). After filtration, the samples were analyzed by HPLC with the same procedure in FIG. 3 to quantify the permeation of desmopressin through each sac. Results are shown in FIG. 4. These data show that the amount of drug that permeated across the epithelium increased when delivered with the expandable device as compared to when administered in the control solution. As a result, the expandable device could promote the transport of drugs through small intestine.

### EXAMPLE 6: In vivo PK study on the 8 mm diameter expandible device

*In vivo* beagle dog experiments were performed in the Centre National de Biologie Expérimentale, Institute National de la Recherche Scientifique (INRS), Laval, Canada. Procedures involving the care and the use of animals in this study were reviewed and approved by the INRS Institutional Animal Care and Use Committee and performed according to the Canadian Council on Animal Care guidelines and policies. Three beagle dogs with body weights around 10 kg were used. The *in vivo* experiments were performed using the expandable device described in Example 4 but containing 1.2 mg desmopressin and 2.0 mg PVP (without any other excipients) loaded in the capsules described in Example 4. As a control formulation, enteric-coated (Eudragit^{™} L100-55, Essen, Germany) capsules as described in Example 4 containing commercial Minirin^{®} drug tablets (6 pieces (0.2 mg of desmopressin per piece) containing 1.2 mg desmopressin in total) were given orally to the dog. After oral administration of the capsules, blood samples were withdrawn at predetermined time points, i.e., 0, 15, 30, 60, 90, 150, 240, and 600 min. Blood samples were collected in K₂EDTA tubes immediately followed by centrifugation at 1700 x g for 10 min at 4 °C. Subsequently, the plasma was stored in clear polypropylene vials at -80°C until further analysis. The drug plasma concentration was evaluated using LC-MS/MS method. Briefly, an aliquot of 900 µL dog plasma sample was mixed with 10 µL internal standard (Goserelin; 25 ng/mL) and 300 µL 4% H3PO4 acidified water into 1.5 mL Eppendorf tubes. The plasma sample was then extracted using Oasis^{™} WAX solid-phase extraction cartridges. After extraction, the solution was evaporated under a low vacuum and reconstituted in 120 µL of 11:11:78 ACN/methanol/DI-water and injected into the Xevo^{™} G2-XS Accurate Mass Spectrometer. Samples were analyzed in TOF-MRM mode with a quantification limit of < 15 pg/mL. Results are shown in FIG. 5. The area under the plasma concentrations vs. time curve (AUC₀₋₆ₕ) for the commercial tablet formulation was 506.21 ± 62.58 pg/mL*h, while the AUC₀₋₆ₕ for the 8mm device was 1825.7 ± 1055.1 pg/mL*h. These data show that the expandable device increased the oral bioavailability of desmopressin by 3.6-fold over the commercial tablets.

### EXAMPLE 7: Effect of the expandable device in combination with a protease inhibitor

*In vivo* experiments were performed on the expandable device described in Example 4 but containing 1.2 mg desmopressin and 2.0 mg PVP. 750 mg of citric acid was loaded together with the 8 mm diameter device in the capsule described in Example 4. As a control formulation, two enteric-coated (Eudragit^{™} L100-55, Essen, Germany) capsules described in Example 4 containing commercial Minirin^{®} drug tablets (3 pieces (0.2 mg per piece) containing 0.6 mg desmopressin in total, and 375 mg citric acid in each capsule) was given orally to the dog. Two capsules were used to fit the 750 mg of citric acid. The drug administration and blood sampling and analysis procedure are the same as described in Example 6. Results are shown in FIG. 6. These data show that the expandable device increased the oral bioavailability by 17.6-fold over the commercial tablets in the presence of citric acid with the AUC₀₋₆ₕ being 54593.4 ± 5497.7 pg/mL*h for the expandable device and 3129.4 ± 2180.0 pg/mL*h for the commercial tablets with citric acid. This difference is significant according to a 2-tailed t test and p value of 0.05.

### EXAMPLE 8: Effect of the expandable device in combination with a permeation enhancer

*In vivo* experiments were performed on the expandable device and enteric capsule described in Example 4 containing 1.2 mg desmopressin, 2.0 mg PVP. 4 mg of a permeation enhancer, i.e., one formulation with C10 and the other with NaTaC, was loaded together with the device in the capsule. The drug administration and blood sampling and analysis procedure are the same as described in Example 6. Results are provided in FIG. 7. The AUC₀₋₆ₕ for the C10 formulation was 3022.3 ± 4337.1 pg/mL*h, while the AUC₀₋₆ₕ for the NaTaC formulation was 1833.0 ± 211.2 pg/mL*h. Therefore, the bioavailability for C10 loaded formulation increased by 6-fold compared to commercial tablets described in Example 6, while the bioavailability for NaTaC loaded formulation increased by 3.6-fold compared to commercial tablets. This difference is significant according to a 2-tailed t test and p value of 0.05.

### EXAMPLE 8: In vivo imaging of the expandable device transit through GI tract

*In vivo* beagle dog X-ray imaging experiments were performed at Shenzhen Advanced Medical Services Co., Ltd, China. Procedures involving the care and the use of animals in this study were reviewed and approved by the company Institutional Animal Care and Use Committee and performed according to the Chinese Animal Welfare Council guidelines and policies. X-ray imaging was performed *in vivo* on beagle dogs to monitor the opening as well as the transit of the expandable device loaded in enteric coated capsules as described in Example 4 but without drug or excipient. through the GI tract. To increase the contrast of the device under X-ray, short gold wires with a diameter of 500 µm and a length of 1.2 mm were embedded inside the elastomer materials forming a ring at the end of each device. After oral administration of the capsule, X-ray images were taken at the same time point as blood sampling, i.e., 0, 15, 30, 60, 90, 150, 240, and 600 min. Results are provided in FIG. 8. These data show that after 30 min, the device was fully expended and migrating along the GI tract. The results show that the deployed expandable device could smoothly transit through the GI tract without disturbing the function of the intestine.

The scope of the claims should not be limited by the embodiments set forth in the examples, but should be given the broadest interpretation consistent with the description as a whole.

### REFERENCES

Dhalla, Arvinder K., Ziad Al-Shamsie, Simret Beraki, Anvesh Dasari, Leonard C. Fung, Laura Fusaro, Anusha Garapaty et al. "A robotic pill for oral delivery of biotherapeutics: safety, tolerability, and performance in healthy subjects." Drug Delivery and Translational Research (2021): 1-12.
Dorkoosh, F.A., Verhoef, J.C., Verheijden, J.H.M., Rafiee-Tehrani M., Borchard, G., Junginger, H.E. Peroral absorption of octreotide in pigs formulated in delivery systems on the basis of superporous hydrogel polymers, Pharm. Res. 19 (2002) 1532-1536.
Granhall C, Donsmark M, Blicher TM, Golor G, Søndergaard FL, Thomsen M, Bækdal TA. Safety and pharmacokinetics of single and multiple ascending doses of the novel oral human GLP-1 analogue, oral semaglutide, in healthy subjects and subjects with Type 2 Diabetes. Clin Pharmacokinet. 2019;58:781-91.
Krauland AH, Guggi D, Bernkop-Schnurch A. (2004). Oral insulin delivery: the potential of thiolated chitosan-insulin tablets on non-diabetic rats. J Control Release, 95: 547-555.
Melmed, R. N., A. A. A. El-Aaser, and S. J. Holt. "Hypertrophy and hyperplasia of the neonatal rat exocrine pancreas induced by orally administered soybean trypsin inhibitor." Biochimica et Biophysica Acta (BBA)-General Subjects 421, no. 2 (1976): 280-288.
Sam, M., Brayden, D. "Formulation strategies to improve the efficacy of intestinal permeation enhancers." Adv. Drug Deliv. Rev (2021): 113925.

## Claims

1. Swallowable, foldable and self-expandable oral drug delivery tubular device, the device:
comprising an outer peripheral surface;
having an expanded length of about 2 cm to about 6 cm;
having an expanded diameter of about 0.45 cm to about 6 cm; and
comprising at least one cavity enclosing a drug, the at least one cavity facing outward from the outer peripheral surface of the device.

2. The device of claim 1, wherein the tubular device comprises a single tubular mesh.

3. The device of claim 1, wherein the tubular device comprises foldable guiding members radially extending from a central tube or rod, each foldable guiding member comprising a guiding member outer peripheral surface, the combined guiding member outer peripheral surfaces of all foldable guiding members forming the device's outer peripheral surface.

4. The device of any one of claims 1-3, wherein the drug is in a formulation further comprising at least one excipient.

5. The device of claim 4, wherein the at least one excipient comprises a permeation enhancer.

6. The device of claim 3 or 4, wherein the at least one excipient comprises a protease inhibitor.

7. The device of any one of claims 1-6, wherein the drug has a molecular weight of about 1 kDa to about 50 kDa.

8. The device of any one of claims 1-6, comprising drug cavities having a volume between 10 nL and 100 µL.

9. Enteric capsule enclosing the device defined in any one of claims 1-8.

10. The capsule of claim 9, further comprising a further excipient.

11. The device of any one of claims 1-8 or the capsule of claim 9 or 10, for oral administration of the drug to a subject.

12. The device of capsule of claim 11, wherein the subject is a human and the device's expanded diameter of about 1.1 cm to about 6 cm.

13. A use of the device as defined in any one of claims 1 to 8 enclosed in an enteric capsule, for the oral administration of the drug to a subject, the use comprising:
orally administering the capsule to the subject, whereby when the capsule releases the device in the small intestine, the device self-expands and positions itself coaxially in the intestine, and
whereby the drug migrates through the intestine epithelium and enters systemic circulation of the subject.

14. The use of claim 13, wherein the subject is a human.
